# EUROPÄISCHE PATENTSCHRIFT

(11) **EP 2 583 285 B1**
(45) Veröffentlichungstag und Bekanntmachung des Hinweises auf die Patenterteilung: **06.07.2016**
(21) Anmeldenummer: 11721737.2
(22) Anmeldetag: 16.05.2011
(51) Int. Cl.: G21K 1/06

(54) **GITTER AUS MINDESTENS ZWEI MATERIALIEN FÜR DIE RÖNTGENBILDGEBUNG**
GRATINGS FOR X-RAY IMAGING, CONSISTING OF AT LEAST TWO MATERIALS
RÉSEAUX CONSTITUÉS D'AU MOINS DEUX MATIÈRES POUR L'IMAGERIE PAR RAYONS X

(30) Priorität: 17.06.2010 DE 102010017426
(43) Veröffentlichungstag der Anmeldung: 24.04.2013
(73) Patentinhaber: Karlsruher Institut Für Technologie (KIT), 73131 Karlsruhe (DE)
(72) Erfinder: MOHR, Jürgen, 75056 Sulzfeld (DE); SCHULZ, Joachim, 76137 Karlsruhe (DE); REZNIKOVA, Elena, Novosibirsk 630087 (RU); PFEIFFER, Franz, 85748 Garching (DE)
(74) Vertreter: Fitzner, Uwe
(86) Internationale Anmeldenummer: PCT/EP2011/057857
(87) Internationale Veröffentlichungsnummer: WO 2011/157500

(56) Entgegenhaltungen:
- DE-A1-102006 037 281
- DE-A1-102008 049 200
- DE-U1-202006 000 330
- FR-A- 830 054
- FR-A1- 2 864 252
- HOLMBERG A ET AL: "Controlled electroplating for high-aspect-ratio zone-plate fabrication", JOURNAL OF VACUUM SCIENCE AND TECHNOLOGY: PART B, AVS / AIP, MELVILLE, NEW YORK, NY, US, Bd. 24, Nr. 6, 31. Oktober 2006 (2006-10-31), Seiten 2592-2596, XP012091707, ISSN: 1071-1023, DOI: DOI:10.1116/1.2362761
- UTSUMI Y ET AL: "SOFT X-RAY W/BE MULTILAYER AND ITS APPLICATION TO A DIFFRACTION GRATING", REVIEW OF SCIENTIFIC INSTRUMENTS, AIP, MELVILLE, NY, US, Bd. 60, Nr. 7, PART 2B, 1. Juli 1989 (1989-07-01), Seiten 2024-2026, XP000038286, ISSN: 0034-6748, DOI: DOI:10.1063/1.1140866
- LINKE JIAN ET AL: "Multilevel microstructures and mold inserts fabricated with planar and oblique x-ray lithography of SU-8 negative photoresist", PROCEEDINGS OF THE SPIE, THE INTERNATIONAL SOCIETY FOR OPTICAL ENGINEERING SPIE, USA, Bd. 4557, 28. September 2001 (2001-09-28), Seiten 69-76, XP002616910, ISSN: 0277-786X, DOI: 10.1117/12.442979

## Beschreibung

Die vorliegende Erfindung betrifft neuartige Gitter für die Röntgenbildgebung mittels Phasenkontrastmethoden und/oder Dunkelfeldbildgebung.

### Stand der Technik:

Die Phasenkontrastbildgebung mit Röntgenstrahlen beruht auf einem gitterbasierten Interferometer, das den Talboteffekt zur Bildgebung nutzt. Dazu werden zwei Gitter senkrecht zum Röntgenstrahl parallel zueinander positioniert. Das Phasengitter g1 besteht aus Linien, die einen Phasenschub von Pi (oder Pi/2) und eine vernachlässigbare Röntgenabsorption verursachen. Die gebeugten Strahlen interferieren gemäß dem fraktionalen Talboteffekt und es entsteht ein Interferenzmuster, das in den zu den Gitterlinien senkrechten Richtungen periodisch ist. Das zweite Gitter dient zur Analyse des Interferenzbildes und soll die Röntgenstrahlung bestmöglich absorbieren [Absorptionsgitter] (Absorption größer 50% ist für eine Bildauswertung mindestens notwendig).

In den Veröffentlichungen Franz Pfeiffer et al., "Phase retrieval and differential phase-contrast imaging with low-brilliance X-ray sources", Nature Physics, Vol. 2, April 2006, Seiten 258-261 und "Hard X-ray dark-field imaging using a grating interferometer", Nature Materials, Vol. 7, Februar 2008, Seiten 134-137, werden die Möglichkeiten der Phasenkontrast-Röntgenbildgebung und der Dunkelfeldbildgebung mit nicht-kohärenten Röntgenquellen beschrieben. Zur Realisierung dieser Bildgebungssysteme müssen Gitterstrukturen mit Perioden im Bereich von wenigen Mikrometern realisiert werden, deren Transmission über den relevanten Energiebereich kleiner als 50% ist. Die relevanten Energien werden durch die jeweilige Anwendung festgelegt. Humanmedizinische Röntgenuntersuchungen des ganzen Körpers werden üblicherweise bei Arbeitsenergienzwischen 50 und 90 keV durchgeführt. Im Falle geringerer Dicke der zu durchdringenden Objekte, z.B. Kinder-CTs, sind oft auch Energien kleiner 50 bis 70 keV von Interesse. So können beispielsweise in der Mammographie kleinere Energien eingesetzt werden. Auch bei Kleintierversuchen ist die Energie geringer. Darüber hinaus ist zu beachten, dass sich bedingt durch die von einer Röntgenröhre abgestrahlten Strahlung auch bei einer hohen "Arbeitsenergie" Strahlung unterhalb der Arbeitsenergie nicht vermeiden lässt.

Heute verwendete Gitter verwenden Gold als Material, das die Röntgenstrahlen stark absorbiert. Wegen der Atomstruktur sackt die Absorption von Gold unterhalb 80.7 keV Röntgenenergie um einen Faktor 4 ab.

Demgemäß führen insbesondere breitbandige Belichtungen mit Röntgenlicht in einem Bereich oberhalb und unterhalb von 80 keV Röntgenenergie nicht zu befriedigenden Ergebnissen.

Bislang ist die Herstellung von Gittern veröffentlicht unter Verwendung von Gold mit dem LIGA-Verfahren (Reznikova, E.; Mohr, J.; Boerner, M.; Nazmov, V.; Jakobs, P.J. Soft X-ray lithography of high aspect ratio SU8 submicron structures, Microsystem Technologies, 14 (2008) p.1683-88 und von konformer Beschichtung von Si-Strukturen mit Gold in einem elektrochemischen Goldbad (C. David, J. Bruder, T. Rohbeck, C. Grünzweig, C. Kottler, A. Diaz, O. Bunk, F. Pfeiffer, Fabrication of diffraction gratings for hard X-ray phase contrast imaging, Microelectric Engineering 84 (2007) 1172-1177), wobei die Si-Strukturen über einen DRIE-Prozess hergestellt werden.

Aus P. Ramm, M. J. Wolf, A. Klumpp, R. Wieland, B. Wunderle, B. Michel Through Silicon Via Technology - Processes and Reliability for Wafer-Level 3D System Integration, Proc. 2008 Electronic Components and Technology Conference pp 841 sind technische Prozesse der Silizium-Technik zur Herstellung von Strukturen mit Wolfram bekannt.

Die Herstellung von vergleichbaren Strukturen aus Blei ist ebenfalls bekannt (V. Lehmann, S. Rönnebeck, MEMS techniques applied to the fabrication of antiscatter grids for X-ray imaging, Sensors and Actuators, A95 (2002), 202-207).

Das Problem sehr geringer Absorption von Gold unterhalb der 80.7 keV-Kante wurde bislang nicht adressiert.

Aus WO 2007/095241 A2 sind Gitter bekannt, die aus zwei Materialien bestehen können, wobei die Materialien nicht in Form von einzelnen Schichten, sondern als Pulvermischungen eingesetzt werden.

Aus DE 10 2006 037 256 A1 sind Phasengitter, die wie der Fachmann weiss, erheblich unterschiedliche Anforderungsprofile als Absorptionsgitter zeigen, bekannt, die aus mehreren Materialien aufgebaut sein können.

Aus US 4,515,876 sind Röntgenlithographiemasken bekannt, jedoch keine Gitter für Röntgenbildgebung.

Weiterhin können als Stand der Technik die folgenden Dokumente angeführt werden: DE 20 2006 000330 U1; Holmberg et al., J. Vac. Sci. Technol. B 24(6),Nov/Dec 2006, Seiten 2592-2596; Utsumi et al., Rev. Sci. Instrum., Vol. 60, No. 7, July 1989, Seiten 2024-2026; FR 2 864 252 A1; DE 10 2006 037281 A1; FR 830 954 A; Linke Jian et al., Proc. SPIE Vol. 4557, 2001, Seiten 69-76; DE 10 2008 049200 A1.

### Aufgabe:

Aufgabe der vorliegenden Erfindung ist es demgemäß, Gitter zur Verfügung zu stellen, die auch unterhalb der Absorptionskante von Gold bei 80.7 keV, insbesondere zwischen 70 und 80 keV, höhere Absorptionskoeffizienten aufweisen, um auch unterhalb von 80.7 keV eine erfolgreiche Abschwächung der Strahlung zu ermöglichen.

Ebenso ist es Aufgabe der vorliegenden Erfindung, Gitter zur Verfügung zu stellen, die für breitbandige Belichtung mit Röntgenlicht, wie es bei einer konventionellen Röntgenröhre der Fall ist, besser geeignet sind als die Gitter des bisherigen Standes der Technik.

Es war weiterhin Aufgabe der vorliegenden Erfindung, Verfahren zur Herstellung solcher Gitter zur Verfügung zu stellen.

Nicht zuletzt war es Aufgabe entsprechende Verwendungsmöglichkeiten zu finden.

### Lösung:

Diese Aufgabe wird durch Gitter umfassend einen Träger und mindestens zwei verschiedene Materialien, sowie durch Verfahren zu deren Herstellung und deren Verwendung gelöst.

### Begriffsdefinitionen:

Die in der vorliegenden Erfindung verwendete Abkürzung "LIGA" steht für "Lithographie, Galvanik und Abformung" und ist ein bekanntes Verfahren zur Herstellung von Mikrostrukturen. Die in der vorliegenden Erfindung verwendete Abkürzung "DRIE" steht für "Deep reactive-ion etching", d.h. reaktives Ionentiefätzen, und ist ein bekanntes Verfahren zur Bearbeitung von Mikrostrukturen. Die in der vorliegenden Erfindung verwendete Abkürzung "CVD" steht für "Chemical Vapor Deposition" und ist ein bekanntes Verfahren zur Stoffabscheidung. Gitter im Sinne der vorliegenden Erfindung sind Gitter zur Analyse des Interferenzbildes in Interferometern für die Röntgenbildgebung mittels Phasenkontrastmethoden und/oder Dunkelfeldbildgebung, insbesondere Absorptionsgitter.

Detaillierte Beschreibung: Absorptionsgitter zur Analyse des Interferenzbildes in Interferometern für die Röntgenbildgebung mittels Phasenkontrastmethoden und/oder Dunkelfeldbildgebung umfassend einen Träger und zwei Röntgenstrahlen absorbierende Materialien, wobei eines der Materialien Gold oder Blei ist, und das zweite Material Wolfram oder Tantal ist, dadurch gekennzeichnet, dass sie eine Schichtstruktur ausgewählt aus der Gruppe bestehend aus Träger/MS1/MS2 oder MS1/ Träger/MS aufweisen, wobei die Bezeichnung MS für Materialschicht steht.

Die vorliegende Erfindung nutzt spezifisch Materialien, deren Absorptionskante für Röntgenstrahlung unterhalb der von Gold liegt. Dies sind alle Materialien mit (nur wenig) geringerer Atom-Ordnungszahl, bis hinunter zu Barium. Durch Verwendung dieser Materialien wird der Energiebereich ab 67.4 keV (Ta) oder 69 keV (W) mit der gleichen Schichtdicke wie Gold um ein mehrfaches besser abgeschwächt.

Um gute Absorptionseigenschaften auch bei breiter Energieverteilung zu erhalten, wird im Rahmen der vorliegenden Erfindung eine Kombination zweier Materialien für die Gitter verwendet, indem z.B. ein Teil des Gitters aus einem ersten Material und der andere Teil aus einem zweiten Material hergestellt wird, wobei eines der Materialien eine Absorptionskante unterhalb derer von Gold aufweist.

Dabei kann in einer Variante das erste Material als Maske im bekannten LIGA-Prozess verwendet werden, um eine in-situ justierte LIGA-Struktur zu erzeugen und damit einen Verbundaufbau zu realisieren.

In einer Variante der vorliegenden Erfindung ist das erste Material Wolfram und das zweite Material Gold.

In einer Variante der vorliegenden Erfindung ist es dabei auch möglich Gold durch Blei zu ersetzen.

Gegenstand der vorliegenden Erfindung ist in einer Variante die Kombination zweier Materialien in Gittern, um ein gegebenes Spektrum spezifisch abschwächen zu können (mit der geforderten Geometrie / Periode des Gitters). Gegenstand der vorliegenden Erfindung ist ferner die Verwendung von Wolfram als Absorbermaterial für die genannten Gitter.

Ebenfalls Gegenstand der vorliegenden Erfindung ist die Verwendung der vorhandenen Technologie "Wolfram-CVD in DRIE Si-Gräben" (siehe P. Ramm et al.), um solche Gitter herzustellen, sowie die Verwendung der vorhandenen Technologie "Bleiguss in geätzte poröse Si-Strukturen (siehe V. Lehmann et al) um solche Gitter herzustellen.

Eine technologische Herausforderung war es, bei der nötigen Kleinheit der Strukturen (um 1 µm Linienbreite), eine ausreichende Absorption zu erzeugen, was eine große Höhe der Linien und damit ein großes Aspektverhältnis erfordert.

Diese geforderte hohe Absorption konnte im Rahmen der vorliegende Erfindung durch die Kombination mindestens zweier Materialien bewältigt werden.

Für den Energiebereich zwischen 69.5 keV und 80.7 keV wird in einer Variante der vorliegenden Erfindung Wolfram angewendet, denn Goldstrukturen müssten etwa 176 µm hoch sein, Wolframstrukturen nur etwa 46 µm (knapp 4-fache Eindringtiefe) um eine Transmission kleiner 50% zu erreichen. Unterhalb von 69.5 keV ist allerdings auch die Absorption von Wolfram relativ gering, so dass dort Gold wieder Vorteile bietet.

Demgemäß ist die Kombination dieser beiden Elemente eine Variante der vorliegenden Erfindung.

Eine weitere Variante der vorliegenden Erfindung ist es, mindestens ein weiteres Material mit einer Absorptionskante bei noch geringerer Energie zusätzlich zu verwenden.

Im Rahmen der vorliegenden Erfindung wurde in einer Variante eine für die Anwendung extrem vorteilhafte spezifische Kombination von Materialien mit unterschiedlicher Absorptionskante und optional zusätzlich angepassten Höhen gefunden, insbesondere durch die Kombination von Gold und Wolfram.

Für breitbandige Anwendungen lassen sich im Rahmen der vorliegenden Erfindung durch eine Anpassung der relativen Höhen von mindestens zwei ausgewählten Materialien, bevorzugt Gold und Wolfram, über den gesamten Energiebereich die Absorptionseigenschaften so einstellen, dass sich eine ausreichende Bildqualität ergibt.

In einer erfindungsgemäß bevorzugten Variante liefert die Kombination aus 100 µm Au und 22 µm W über den gesamten Energiebereich eine Absorption von besser 50%. Damit kann in der medizinischen Analyse die Röntgenenergie auch im Bereich von 70 bis 80 keV so gewählt werden, dass die zu untersuchenden Gewebeproben möglichst sensitiv dargestellt werden.

Prinzipiell ist die Herstellung von Gittern mit geringer Breite bis zu 1 µm in Gold mit Höhen von bis zu 100 µm möglich, so dass oberhalb von 80.7 keV bis ca. 110 keV die Transmission deutlich unter 50% liegt. Größere Dicken sind aber aus heutiger Sicht nur schwer zu realisieren, da die Stabilität der Form mit der dritten Potenz der Höhe abnimmt und kleinste Belastungen zu einer Verbiegung der Stege führt.

Im Bereich zwischen 69.5 und 80.7 keV kann dagegen eine Transmission kleiner 50% durch eine ca. 46 µm dicke Wolframstruktur erreicht werden. Problematisch ist allerdings, dass die Transmission dieser Wolframstruktur in den anderen Energiebereichen höher ist und somit dort ein schlechteres Gitter darstellt. Zudem sind keine Verfahren bekannt, die die Strukturierung von Wolfram mit Mikrometerabmessungen und Aspektverhältnis von 100 ermöglicht.

Die Problematik der hohen Transmission im Falle von Gold im Bereich zwischen 69.5 und 80.7 keV kann unter Beibehaltung einer hohen Absorption außerhalb dieses Bereiches durch die vorliegende Erfindung, nämlich die Kombination verschiedener Materialien, gelöst werden.

Die vorliegende Erfindung betrifft demgemäß insbesondere Gitter umfassend einen Träger und mindestens zwei Materialien, insbesondere Metalle.

Dabei bedeutet Schicht im Rahmen der vorliegenden Erfindung nicht zwangsweise eine planare, ebene Fläche. Die Gitter der vorliegenden Erfindung beruhen z.B. von der Seite gesehen, auf kammartigen Strukturen. Demgemäß kann eine Schicht aus nebeneinander angeordneten Lamellen oder aus von der Seite gesehen kammartigen Strukturen (entsprechend Bodenplatte *und* Zinken bzw. Lamellen) bestehen, wohingegen andere Schichten unabhängig davon ebene Platten sein können.

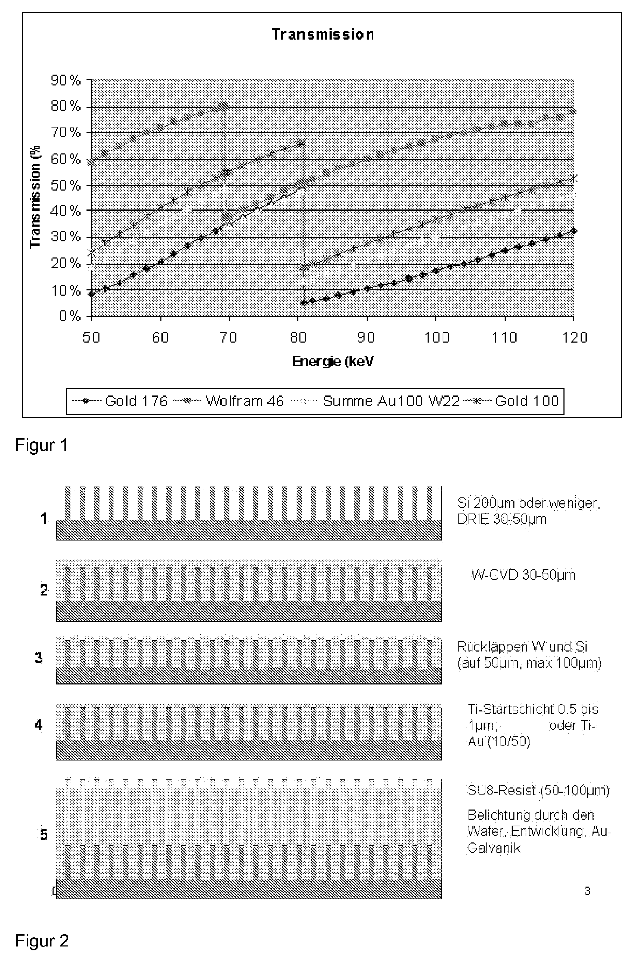

Dies ist dem Fachmann hinreichend bekannt und ergibt sich bei den erfindungsgemäßen Verfahren auch ausgehend von den angewendeten Techniken zwangsläufig (vgl. auch Figur 2).

Eine Variante der vorliegenden Erfindung sind dabei Gitter die aus einem Träger und genau zwei Metallen bestehen, wobei die Metalle bevorzugt Gold und Wolfram sind.

Eine weitere Variante der vorliegenden Erfindung sind dabei Gitter mit der Schichtstruktur Träger/Au/W oder Träger/W/Au oder W/Träger/Au, bevorzugt Träger/W/Au.

Eine Variante der vorliegenden Erfindung sind dabei Gitter die aus einem Träger und genau drei Metallen bestehen, wobei zwei der Metalle bevorzugt Gold und Wolfram sind.

Die Träger der erfindungsgemäßen Gitter sind in einer Variante ausgewählt aus der Gruppe von Materialien mit geringer Absorption.

Unter einer geringen Absorption bei dem Trägermaterial wird verstanden, dass die Absorption gegenüber der Absorption durch die Metalle nur geringfügig ist. Diese Eigenschaft des Trägermaterials ist dem Fachmann geläufig und kann gegebenenfalls, soweit ihm nicht ohnehin schon bekannt, in einfacher Weise aus Tabellenwerken entnommen werden.

Es eignen sich bevorzugt alle Metalle bis zur Kernladungszahl 30 in geeignet ausgewählter Schichtdicke.

In einer Variante bestehen die Träger aus Silizium und/oder dessen Verbindungen wie insbesondere Siliziumnitrid, bevorzugt Silizium.

In einer anderen Variante bestehen die Träger aus Polymeren, insbesondere Polyimiden,

In einer weiteren Variante bestehen die Träger aus Metallen mit niedriger Kernladungszahl von kleiner oder gleich 30, insbesondere Titan.

In einer zusätzlichen Variante bestehen die Träger aus einer Kombination von Silizium bzw. Siliziumverbindungen, Polymeren und/oder Materialien mit niedriger Kernladungszahl.

Insbesondere kann durch eine Kombination aus 22 µm Wolfram und 100 µm Gold die Transmission im Bereich von 69.5 bis 80.7 keV gegenüber einer 100 µm dicken Goldstruktur um mehr als 10% (ca. 16%) auf unter 50% gesenkt werden. In den anderen Bereichen ist sie etwa 5% geringer als die Transmission des reinen 100 µm dicken Goldes.

Für den Fall von 100 µm Gold und 22 µm Wolfram liegt sie für den gesamten Energiebereich darunter.

Besonders bevorzugte Ausgestaltungen der vorliegenden Erfindung sind Kombinationen aus Au und W im Höhenverhältnis von 2:1 bis 6:1. Erfindungsgemäße Beispiele sind
- 100 µm Au und 22 µm W
- 90 µm Au und 30 µm W
- 80 µm Au und 40 µm W

Als absorbierende Materialien können außerhalb des Rahmens der vorliegenden Erfindung neben der Kombination aus W und Au in Varianten auch die folgenden Materialien kombiniert werden: Pb/Pt, Pb/W, Pb/Ta, Au/Ta, Au/Ba, Au/BaF₂, Au/Gd₂O₂S

Eine erfindungsgemäße Variante zur Herstellung der erfindungsgemäßen Gitter mit zwei Materialien, insbesondere Metallen, ist ein Verfahren I) umfassend die Schritte, bevorzugt bestehend aus den Schritten:
a1) In eine Trägerscheibe, bevorzugt eine Siliziumscheibe, wird die Gitterstruktur eingearbeitet, bevorzugt durch DRIE geätzt,
b1) diese Gitterstrukturen werden mit einem ersten Material, bevorzugt W, befüllt, bevorzugt über CVD,
c1) auf der Oberfläche abgeschiedenes erstes Material, bevorzugt W, wird entfernt, bevorzugt durch Rücksputtern, und der Wafer, bevorzugt Siliziumwafer, auf der Rückseite zurückgedünnt, bevorzugt durch einen Läppprozeß, und gegebenenfalls zur mechanischen Stabilisierung auf einen Rahmen aufgespannt,
d1) auf die Vorderseite (Verbundstruktur erstes Material/Wafer) wird eine metallische Schicht aufgebracht, die später als Galvanikstartschicht dient,
e1) auf dieser Schicht wird ein Negativresist aufgebracht, der
f1) gemäß dem LIGA-Verfahren mit Röntgenstrahlung von der Rückseite strukturiert wird,
g1) die frei entwickelten Gräben werden mit einem zweiten Material befüllt.

Dabei dient bei dieser Variante die erste Material/Waferstruktur als Maske für den Negativresist, d.h. der eingedünnte Wafer wird von der Rückseite durchstrahlt. Die Strukturen des ersten Materials stellen den Absorber dar und erlauben die Erzeugung von Grabenstrukturen im Negativresist. Diese werden galvanisch mit dem zweiten Material, bevorzugt Au, befüllt. Durch die integrierte Maske des ersten Metalls kommen die Strukturen des zweiten Materials ohne weitere Justierung über den Strukturen des ersten Materials zum Liegen. Die Begriffe Wafer und Trägerscheibe sind dabei synonym.

Alternativ sind die Ausgestaltungen
d1.2) auf die Rückseite (Verbundstruktur erstes Material/Wafer) wird eine metallische Schicht aufgebracht, die später als Galvanikstartschicht dient,
   und
f1.2) gemäß dem LIGA-Verfahren mit Röntgenstrahlung von der Vorderseite strukturiert wird
für dieses Herstellungsverfahren möglich.

Eine zweite erfindungsgemäße Variante zur Herstellung der erfindungsgemäßen Gitter mit zwei Materialien, insbesondere Metallen, ist ein Verfahren II) umfassend die Schritte, bevorzugt bestehend aus den Schritten:
a2) entsprechend a1)
b2) entsprechend b1)
c2) entsprechend c1)
d2) entsprechend d1) bzw. d1.2)
e2) auf dieser Schicht wird ein Positivresist aufgebracht, der
f2) über eine justierte Maske gemäß dem LIGA-Verfahren mit Röntgenstrahlung von der Vorderseite oder auch der Rückseite strukturiert wird,
g2) die frei entwickelten Gräben werden mit einem zweiten Material befüllt.

Durch diese beiden Verfahren I) und II) lassen sich sehr gut Verbundstrukturen, insbesondere Gitter, aus einem ersten und einem zweiten Material, bevorzugt Wolfram und Gold, erzeugen.

In Varianten der Verfahren I) und II) der vorliegenden Erfindung werden folgende Merkmale angewendet, wobei die verschiedenen jeweiligen Bereiche der einzelnen Merkmale unabhängig von beliebigen Bereichen anderer Merkmale sind (d.h. es ist beispielsweise möglich die höchst bevorzugten Bereiche einzelner Schritte mit den breiteren Bereichen anderer Schritte zu kombinieren):
a1), a2) Siliziumschichtdicke von 150 bis 1000 µm bevorzugt von 500 bis 800 µm, insbesondere von 500 bis 700 µm oder von 180 bis 220 µm
a1), a2) DRIE bis zu einer Tiefe von 20 bis 100 µm bevorzugt von 30 bis 70 µm insbesondere von 30 bis 50 µm
b1), b2) konforme Beschichtungsdicke von 0.5 bis 6 µm, bevorzugt von 1 bis 3 µm
c1), c2) Rückläppen auf maximal 100 µm, bevorzugt maximal 70 µm, insbesondere maximal 50 µm
d1), d2) Materialschichtdicke von 0,05 bis 10 µm, bevorzugt von 0,3 bis 3 µm, insbesondere von 1 bis 3 µm
d1), d2) Material der metallischen Schicht ausgewählt aus der Gruppe bestehend aus Ti, Cr, Ti/Au, Ti/Au, Cr/Au, Cu, Ag und Mischungen davon,
e1) Schichtdicke Negativresist von 30 bis 1000 µm, bevorzugt von 40 bis 200 µm insbesondere von 50 bis 150 µm
e1) Negativresist ausgewählt aus der Gruppe bestehend aus chemisch verstärkten Resists, insbesondere Epoxid-basierten Resists, Polyimiden und Mischungen davon
e2) Schichtdicke Positivresist von 30 bis 1000 µm, bevorzugt von 40 bis 200 µm insbesondere von 50 bis 150 µm
e2) Positivresist ausgewählt aus der Gruppe bestehend aus Acrylaten, Phenolharzen und Mischungen davon.

Als Träger bzw. Wafer kommen die oben bereits als Träger beschriebenen Substanzen zum Einsatz.

An diese Verfahren anschließend lassen sich weitere Schichten mit den entsprechenden Verfahrensschritten aufbringen.

Entsprechende Verfahren sind ebenfalls Gegenstand der vorliegenden Erfindung.

Alternativ den beiden Verfahren I) und II) kann die Struktur des zweiten Metalls auch auf der Rückseite des eingedünnten Substrates hergestellt werden. In diesem Fall wird nach dem Eindünnen des Wafers dieser auf einen Rahmen aufgebracht, so dass die Unterseite des Wafers offen zu liegen kommt. Diese wird dann mit dem Negativlack beschichtet und der Lithographie- und Galvanikprozess wird entsprechend durchgeführt. Der Vorteil dieser Variante liegt darin, dass der Silizium deutlich unter 50 µm eingedünnt werden kann, sofern es aus röntgenlithographischen Gründen notwendig ist; dies entspricht demgemäß Verfahren III)
umfassend die Schritte, bevorzugt bestehend aus den Schritten:
a3) In eine Trägerscheibe werden die Gitterstrukturen eingearbeitet,
b3) diese Gitterstrukturen werden mit einem ersten Metall befüllt,
c3) das auf der Oberfläche abgeschiedene erste Metall wird durch einen Läppprozess entfernt,
d3) der Wafer wird auf einen Rahmen aufgebracht, so dass die Unterseite des Wafers offen zu liegen kommt, der Wafer auf der Rückseite zurückgedünnt und eine metallische Schicht aufgebracht wird,
e3) auf dieser Unterseite wird ein Negativlack aufgebracht, der
f3) gemäß dem LIGA-Verfahren strukturiert wird,
g3) die frei entwickelten Gräben werden mit einem zweiten Material befüllt.

Alternativ kann in Schritt e3) ein Positivlack aufgebracht und dann gemäß dem LIGA-Verfahren über eine Maske justiert zur ersten Struktur bestrahlt werden. Auch durch dieses Verfahren III) lassen sich sehr gut Verbundstrukturen, insbesondere Gitter, aus einem ersten und einem zweiten Material, bevorzugt Wolfram und Gold, erzeugen.

Auch an dieses Verfahren anschließend lassen sich weitere Schichten mit den entsprechenden Verfahrensschritten aufbringen.

Entsprechende Verfahren sind ebenfalls Gegenstand der vorliegenden Erfindung.

Die erfindungsgemäßen Gitter können verwendet werden in röntgenbasierten Systemen der Medizintechnik (z.B. Röntgentomographie), bei der zerstörungsfreien Prüfung von Materialien und Komponenten, im Pharmascreening, in Gepäck- und Briefscannern, in Gantry-Einheiten von Computertomographen, bei der Röntgenoptik in Mikro-CT-Geräten, in röntgenoptischen Systemen für die medizinische Radiographie (Mammographie) bzw. Angiographie, bei Tumor-Therapie-Untersuchungen bei Kleintieren, in der Röntgenbildgebung im weitesten Sinn (Tomographie, Radiographie, Streulichtgitter, Materialuntersuchungen).

Die verschiedenen Ausgestaltungen der vorliegenden Erfindung, z.B. aber nicht ausschließlich diejenigen der verschiedenen abhängigen Ansprüche, können dabei in beliebiger Art und Weise miteinander kombiniert werden.

### Figurenbeschreibung:

Die Figuren sind nicht maßstabsgetreu. Aus Gründen der Klarheit und zur einfacheren Darstellung können einige Merkmale der Erfindung übertrieben groß oder in schematischer Form dargestellt sein, ebenso können demgemäß einige Details von konventionellen bzw. bekannten Elementen nicht dargestellt sein.

### Figur 1:

Figur 1 zeigt, dass bei Strukturen in Gold mit Dicken von 100 µm oberhalb von 80 keV bis ca. 110 keV die Transmission deutlich unter 50% liegt (lila Kurve; sternchenförmige Symbole). Wie zu erkennen, steigt die Transmission unterhalb von 80 keV sprunghaft an und beträgt sogar mehr als 60%.

Weiterhin ist aus Figur 1 ersichtlich, dass im Bereich zwischen 70 und 80 keV eine Transmission kleiner 50% durch eine 46 µm dicke Wolframstruktur erreicht werden kann (rosa Kurve; quadratische Symbole in Figur 1), wobei allerdings die Transmission dieser Wolframstruktur in den anderen Energiebereichen höher ist. Die gelbe Kurve; dreieckige Symbole zeigt, dass eine Kombination aus 22 µm Wolfram und 100 µm Gold die Transmission im Bereich von 70 bis 80 keV gegenüber einer 100 µm Goldstruktur um mehr als 10% auf unter 50% senkt. In den anderen Bereichen ist sie um etwa 5% geringer, als die Transmission des reinen 100 µm dicken Goldes.

Ferner ist zu ersehen, dass erst eine Erhöhung der Schichtdicke des Goldes auf 176 µm (blaue Kurve, karoförmige Symbole) die Transmission im gesamten Energiebereich auf unter 50% senkt.

### Figur 2:

Figur 2 illustriert die Herstellung von erfindungsgemäßen Gittern auf Basis der Kombination zweier Metalle, gemäß der erfindungsgemäßen Variante I).

## Patentansprüche

1. Absorptionsgitter zur Analyse des Interferenzbildes in Interferometern für die Röntgenbildgebung mittels Phasenkontrastmethoden und/oder Dunkelfeldbildgebung umfassend einen Träger und zwei Röntgenstrahlen absorbierende Materialien,
wobei eines der Materialien Gold oder Blei ist,
und das zweite Material Wolfram oder Tantal ist
**dadurch gekennzeichnet, dass** sie eine Schichtstruktur ausgewählt aus der Gruppe bestehend aus
Träger/MS1/MS2,
oder
MS1/Träger/MS2,
aufweisen,
wobei die Bezeichnung MS für Materialschicht steht.

2. Absorptionsgitter nach Anspruch 1, **dadurch gekennzeichnet, dass** die Materialien Gold und Wolfram sind.

3. Absorptionsgitter nach Anspruch 2, **dadurch gekennzeichnet, dass** die Schichtdicke von W zur Schichtdicke von Au im Verhältnis 1:2 bis 1:6 steht.

4. Absorptionsgitter nach Anspruch 3, **dadurch gekennzeichnet, dass** die Schichtstruktur Träger/Au/W oder Träger/W/Au oder W/Träger/Au ist.

5. Absorptionsgitter nach einem der Ansprüche 1 bis 4, **dadurch gekennzeichnet, dass** der Träger ausgewählt ist aus der Gruppe bestehend Silizium bzw. Siliciumverbindungen, Polymeren und/oder Materialien mit niedriger Kernladungszahl oder einer Kombination davon.

6. Verfahren zur Herstellung der Absorptionsgitter nach einem der Ansprüche 1 bis 5 umfassend die Schritte:
a1) In eine Trägerscheibe wird die Gitterstruktur eingearbeitet,
b1) diese Gitterstrukturen werden mit einem ersten Material befüllt,
c1) auf der Oberfläche abgeschiedenes erstes Material wird entfernt und die Trägerscheibe auf der Rückseite zurückgedünnt,
d1.1) auf die Vorderseite (Verbundstruktur erstes Material/Trägerscheibe) oder
d 1.2) auf die Rückseite (Verbundstruktur erstes Material/Trägerscheibe) wird eine metallische Schicht aufgebracht, die später als Galvanikstartschicht dient,
e1) auf dieser Schicht wird ein Negativresist aufgebracht, der gemäß dem LIGA-Verfahren mit Röntgenstrahlung
f1.1) von der Vorderseite oder
f1.2) von der Rückseite strukturiert wird,
g1) die frei entwickelten Gräben werden mit einem zweiten Material befüllt.

7. Verfahren II) zur Herstellung der Absorptionsgitter nach einem der Ansprüche 1 bis 5 umfassend die Schritte:
a2) In eine Trägerscheibe wird die Gitterstruktur eingearbeitet,
b2) diese Gitterstrukturen werden mit einem ersten Material befüllt,
c2) auf der Oberfläche abgeschiedenes erstes Material wird entfernt und die Trägerscheibe auf der Rückseite zurückgedünnt,
d2.1) auf die Vorderseite (Verbundstruktur erstes Material/Trägerscheibe) oder
d2.2) auf die Rückseite (Verbundstruktur erstes Material/Trägerscheibe) wird eine metallische Schicht aufgebracht, die später als Galvanikstartschicht dient,
e2) auf dieser Schicht wird ein Positivresist aufgebracht, der
f2) über eine justierte Maske gemäß dem LIGA-Verfahren mit Röntgenstrahlung von der Vorderseite oder Rückseite strukturiert wird
g2) die frei entwickelten Gräben werden mit einem zweiten Material befüllt.

8. Verfahren zur Herstellung der Absorptionsgitter nach einem der Ansprüche 1 bis 5 umfassend die Schritte:
a3) In eine Trägerscheibe werden die Gitterstrukturen eingearbeitet,
b3) diese Gitterstrukturen werden mit einem ersten Metall befüllt,
c3) das auf der Oberfläche abgeschiedene erste Metall wird durch einen Läppprozess entfernt,
d3) die Trägerscheibe wird auf einen Rahmen aufgebracht, so dass die Unterseite der Trägerscheibe offen zu liegen kommt, die Trägerscheibe auf der Rückseite zurückgedünnt und eine metallische Schicht aufgebracht wird,
e3) auf dieser Unterseite wird ein Negativlack aufgebracht der
f3) gemäß dem LIGA-Verfahren strukturiert wird,
g3) die frei entwickelten Gräben werden mit einem zweiten Material befüllt.

9. Verfahren nach Anspruch 8, **dadurch gekennzeichnet, dass** in Schritt e3) ein Positivresist aufgebracht wird und dieser gemäß dem LIGA-Verfahren über eine Maske justiert zur ersten Struktur bestrahlt wird.

10. Verwendung der Absorptionsgitter nach einem der Ansprüche 1 bis 5 in röntgenbasierten Systemen der Medizintechnik (z.B. Röntgentomographie), bei der zerstörungsfreien Prüfung von Materialien und Komponenten, im Pharmascreening, in Gepäck- und Briefscannern, in Gantry-Einheiten von Computertomographen, bei der Röntgenoptik in Mikro-CT-Geräten, in röntgenoptischen Systemen für die medizinische Radiographie (Mammographie) bzw. Angiographie, bei Tumor-Therapie-Untersuchungen bei Kleintieren, in der Röntgenloildgebung im weitesten Sinn, wie Tomographie, Radiographie, Streulichtscheiben, Materialuntersuchungen.

## Claims

1. Gratings for the analysis of the interference image in interferometers for the x-ray imaging via phase-contrast methods and/or dark field imaging comprising a carrier and two materials which absorb X-rays,
wherein one of the materials is gold or lead,
and the second material is tungsten or tantalum,
**characterized in that** they
have a layer structure selected from the group consisting of
carrier/ML1/ML2
or
ML1/carrier/ML2,
wherein the designation ML stands for material layer.

2. The gratings as claimed in claim 1, **characterized in that** the materials are gold and tungsten.

3. The gratings as claimed in claim 2, **characterized in that** the layer thickness of W is in a ratio of 1:2 to 1:6 relative to the layer thickness of Au.

4. The gratings as claimed in claim 3, **characterized in that** the layer structure is carrier/Au/W or carrier/W/Au or W/carrier/Au.

5. The gratings as claimed in any of claims 1 to 4, **characterized in that** the carrier is selected from the group consisting of silicon or silicon compounds, polymers and/or materials having a low atomic number or a combination thereof.

6. A method for producing the gratings as claimed in any of claims 1 to 5 comprising the following steps:
a1) the grating structure is worked into a carrier wafer,
b1) these grating structures are filled with a first material,
c1) first material deposited on the surface is removed and the carrier wafer is thinned back on the rear side,
d1.1) onto the front side (composite structure first material/carrier wafer) or
d1.2) onto the rear side (composite structure first material/carrier wafer) a metallic layer is applied, which later serves as an electroplating start layer,
e1) a negative resist is applied on said layer and is patterned in accordance with the LIGA method with X-ray radiation
f1.1) from the front side or
f1.2) from the rear side,
g1) and the freely developed trenches are filled with a second material.

7. A method II) for producing the gratings as claimed in any of claims 1 to 5 comprising the following steps:
a2) the grating structure is worked into a carrier wafer,
b2) these grating structures are filled with a first material,
c2) first material deposited on the surface is removed and the carrier wafer is thinned back on the rear side,
d2.1) onto the front side (composite structure first material/carrier wafer) or
d2.2) onto the rear side (composite structure first material/carrier wafer) a metallic layer is applied, which later serves as an electroplating start layer,
e2) a positive resist is applied on said layer and
f2) is patterned via an aligned mask in accordance with the LIGA method with X-ray radiation from the front side or rear side
g2) and the freely developed trenches are filled with a second material.

8. A method for producing the gratings as claimed in any of claims 1 to 5, comprising the following steps:
a3) the grating structures are worked into a carrier wafer,
b3) these grating structures are filled with a first metal,
c3) the first metal deposited on the surface is removed by a lapping process,
d3) the carrier wafer is applied to a frame, such that the underside of the carrier wafer is exposed, the carrier wafer is thinned back on the rear side and a metallic layer is applied,
e3) a negative resist is applied on said underside and
f3) is patterned in accordance with the LIGA method,
g3) and the freely developed trenches are filled with a second material.

9. The method as claimed in claim 8, **characterized in that** in step e3) a positive resist is applied and is irradiated in accordance with the LIGA method via a mask in a manner aligned with respect to the first structure.

10. The use of the gratings as claimed in any of claims 1 to 5 in X-ray-based systems appertaining to medical technology (e.g. X-ray tomography), in the non-destructive testing of materials and components, in pharmaceutical screening, in baggage and mail scanners, in gantry units of computer tomographs, in X-ray optics in micro-CT apparatuses, in X-ray optical systems for medical radiography (mammography) or angiography, in tumor therapy examinations for small animals, in X-ray imaging in the broadest sense, such as tomography, radiography, light diffuser screens, material investigations.

## Revendications

1. Réseau d'absorption pour l'analyse d'une image d'interférence dans des interféromètres pour l'imagerie aux rayons X par des méthodes de contraste de phase et/ou une imagerie sur fond noir, comprenant un support et deux matériaux absorbant les rayons X,
l'un des matériaux étant l'or ou le plomb,
et le deuxième matériau étant le tungstène ou le tantale,
**caractérisé en ce qu'**ils présentent une structure de couches, choisie dans les groupes consistant en Support/MS1/MS2,
ou
MS1/support/MS2,
la désignation MS représentant une couche de matériau.

2. Réseau d'absorption selon la revendication 1, **caractérisé en ce que** les matériaux sont l'or et le tungstène.

3. Réseau d'absorption selon la revendication 2, **caractérisé en ce que** l'épaisseur de couche de W, rapportée à l'épaisseur de couche d'Au, correspond à un rapport de 1:2 à 1:6.

4. Réseau d'absorption selon la revendication 3, **caractérisé en ce que** la structure des couches est support/Au/W ou support/W/Au ou W/support/Au.

5. Réseau d'absorption selon l'une des revendications 1 à 4, **caractérisé en ce que** le support est choisi dans le groupe comprenant le silicium ou les composés du silicium, les polymères et/ou les matériaux à faible numéro atomique, ou une combinaison de ceux-ci.

6. Procédé de fabrication d'un réseau d'absorption selon l'une des revendications 1 à 5, comprenant les étapes :
a1) on incorpore la structure des réseaux dans un disque formant support,
b1) on remplit ces structures de réseau d'un premier matériau,
c1) on élimine le premier matériau déposé sur la surface, et on amincit sur la face arrière le disque formant support,
d1.1) on applique sur la face avant (structure composite premier matériau/disque formant support) ou
d1.2) sur la face arrière (structure composite premier matériau/disque formant support), une couche métallique qui sert ultérieurement de couche de départ pour dépôt électrolytique,
e1) on applique sur cette couche une réserve négative, qui est structurée, par le procédé LIGA, avec un rayonnement X
f1.1) provenant de la face avant, ou
f1.2) provenant de la face arrière,
g1) on remplit d'un deuxième matériau les rainures qui se développent librement.

7. Procédé II) pour la fabrication d'un réseau d'absorption selon l'une des revendications 1 à 5, comprenant les étapes :
a2) on incorpore la structure de réseau dans un disque formant support,
b2) on remplit d'un premier matériau ces structures de réseau,
c2) on élimine le premier matériau qui s'est déposé sur la surface, et on amincit le disque formant support sur la face arrière,
d2.1) on applique sur la face avant (structure composite premier matériau/disque formant support) ou
d2.2) sur la face arrière (structure composite premier matériau/disque formant support), une couche métallique qui sert ultérieurement de couche de départ pour le dépôt électrolytique,
e2) on applique sur cette couche une réserve positive,
f2) qui est structurée par l'intermédiaire d'un masque ajusté par le procédé LIGA avec un rayonnement X provenant de la face avant ou de la face arrière,
g2) on remplit d'un deuxième matériau les rainures qui se sont développées librement.

8. Procédé de fabrication d'un réseau d'absorption selon l'une des revendications 1 à 5, comprenant les étapes :
a3) on incorpore les structures de réseau dans un disque formant support,
b3) on remplit d'un premier métal ces structures de réseau,
c3) on élimine par un procédé par abrasion le premier métal qui s'est déposé sur la surface,
d3) on applique les disques formant support sur un cadre de telle sorte que la face inférieure du disque formant support devient ouverte, on amincit le disque formant support sur la face arrière, et on applique une couche métallique,
e3) on applique sur cette face inférieure un vernis négatif,
f3) qui est structuré par le procédé LIGA,
g3) on remplit d'un deuxième matériau les rainures qui se sont développés librement.

9. Procédé selon la revendication 8, **caractérisé en ce qu'**on applique dans l'étape e3) une réserve positive, et on irradie cette dernière par le procédé LIGA par l'intermédiaire d'un masque ajusté à la première structure.

10. Utilisation des réseaux d'absorption selon l'une des revendications 1 à 5 dans des systèmes de technique médicale se fondant sur les rayons X (par exemple la tomographie aux rayons X), lors de l'essai non destructif de matériaux et composants, lors du criblage de produits pharmaceutiques, dans les scanners pour colis et lettres, dans les unités à portique des équipements de tomodensitométrie, en optique des rayons X dans les équipements de microtornadensitométrie, dans les systèmes optiques à rayons X pour la radiographie médicale (mammographie) ou l'angiographie, lors des examens pour le traitement des tumeurs des petits animaux, en imagerie aux rayons X dans son sens le plus large, comme la tomographie, la radiographie, les disques à écran diffusant, les études des matériaux.
